# FASCICULE DE BREVET EUROPEEN

(11) **EP 0 841 341 B1**
(45) Date de publication et mention de la délivrance du brevet: **07.04.1999**
(21) Numéro de dépôt: 97402555.3
(22) Date de dépôt: 28.10.1997
(51) Int. Cl.: C07F 7/08, C07F 7/21, A61K 7/42

(54) **Nouveaux filtres solaires, compositions cosmétiques photoprotectrices les contenant et utilisations**
Sonnenschutzfilter, und diese enthaltende kosmetische Sonnenschutzzusammensetzungen
Sunscreen agents, and cosmetic compositions containing them

(30) Priorité: 08.11.1996 FR 9613684
(43) Date de publication de la demande: 13.05.1998
(73) Titulaire: L'OREAL, 75008 Paris (FR)
(72) Inventeur: Richard, Hervé, 93240 Villepinte (FR); Leduc, Madeleine, 75011 Paris (FR)
(74) Mandataire: Miszputen, Laurent

(56) Documents cités:
- EP-A- 0 507 691
- EP-A- 0 517 104
- EP-A- 0 775 698

## Description

L'invention concerne de nouveaux dérivés de s-triazine substitués par au moins un groupement siliconé et au moins un groupement aminobenzylidène camphre, aminobenzalmalonate, aminobenzoate, ou encore aminobenzotriazole, aminobenzimidazole, aminobenzoxazole, aminobenzothiazole, aminosalicylate, aminocinnamate ou/et amino cinnamonitrile, ces composés étant plus particulièrement utilisables à titre de filtres organiques solaires dans des compositions cosmétiques destinées à la protection de la peau et des cheveux contre le rayonnement ultraviolet. L'invention concerne également l'utilisation desdits composés dans l'application cosmétique susmentionnée, ainsi que les compositions cosmétiques à propriétés améliorées les contenant.

On sait que les radiations lumineuses de longueurs d'onde comprises entre 280 nm et 400 nm permettent le brunissement de l'épiderme humain, et que les rayons de longueurs d'onde plus particulièrement comprises entre 280 et 320 nm, connus sous la dénomination d'UV-B, provoquent des érythèmes et des brûlures cutanées qui peuvent nuire au développement du bronzage naturel. Pour ces raisons ainsi que pour des raisons esthétiques, il existe une demande constante de moyens de contrôle de ce bronzage naturel en vue de contrôler ainsi la couleur de la peau ; il convient donc de filtrer ce rayonnement UV-B.

On sait également que les rayons UV-A, de longueurs d'onde comprises entre 320 et 400 nm, qui provoquent le brunissement de la peau, sont susceptibles d'induire une altération de celle-ci, notamment dans le cas d'une peau sensible ou d'une peau continuellement exposée au rayonnement solaire. Les rayons UV-A provoquent en particulier une perte d'élasticité de la peau et l'apparition de rides conduisant à un vieillissement cutané prématuré. Ils favorisent le déclenchement de la réaction érythémateuse ou amplifient cette réaction chez certains sujets et peuvent même être à l'origine de réactions phototoxiques ou photo-allergiques. Ainsi, pour des raisons esthétiques et cosmétiques telles que la conservation de l'élasticité naturelle de la peau par exemple, de plus en plus de gens désirent contrôler l'effet des rayons UV-A sur leur peau. Il est donc souhaitable de filtrer aussi le rayonnement UV-A.

De nombreux composés destinés à la photoprotection (UV-A et/ou UV-B) de la peau ont été proposées à ce jour.

La plupart d'entre eux sont des composés aromatiques présentant une absorption des rayons UV dans la zone comprise entre 280 et 315 nm, ou dans la zone comprise entre 315 et 400 nm ou bien encore dans l'ensemble de ces deux zones. Ils sont le plus souvent formulés dans des compositions antisolaires qui se présentent sous la forme d'une émulsion de type huile-dans-eau (c'est à dire un support cosmétiquement acceptable constitué d'une phase continue dispersante aqueuse et d'une phase discontinue dispersée huileuse) et qui contiennent donc, à des concentrations diverses, un ou plusieurs filtres organiques classiques à fonction aromatique, lipophiles et/ou hydrophiles, capables d'absorber sélectivement les rayonnements UV nocifs, ces filtres (et leurs quantités) étant sélectionnés en fonction du facteur de protection solaire recherché (le facteur de protection solaire s'exprimant mathématiquement par le rapport du temps d'irradiation nécessaire pour atteindre le seuil érythématogène avec le filtre UV au temps nécessaire pour atteindre le seuil érythématogène sans filtre UV).

Outre leur pouvoir filtrant, ces composés à activité anti-UV doivent également présenter de bonnes propriétés cosmétiques dans les compositions qui les contiennent, une bonne solubilité dans les solvants usuels et en particulier les corps gras tels que les huiles et les graisses, ainsi qu'une bonne résistance à l'eau et à la transpiration (rémanence).

Parmi tous les composés aromatiques qui ont été préconisés à cet effet, on peut citer notamment les dérivés de l'acide p- aminobenzoïque, les dérivés du benzylidènecamphre, les dérivés de l'acide cinnamique et les dérivés du benzalmalonate. Cependant, certaines de ces substances ne présentent pas toutes les propriétés requises pour une utilisation convenable comme filtres UV dans les compositions antisolaires. En particulier, leur pouvoir filtrant intrinsèque peut être insuffisant, leur solubilité dans les différents types de formulations utilisés en matière de protection solaire n'est pas toujours suffisamment bonne (liposolubilité notamment), elles peuvent ne pas posséder une stabilité suffisante à la lumière (photostabilité) et elles peuvent également présenter une mauvaise résistance à l'eau et à la sueur. Il est également souhaitable que ces substances filtrantes ne pénètrent pas dans la peau.

Ainsi, dans le cas plus particulier des substances filtres de type benzalmalonate, benzotriazole ou benzylidène camphre, on a cherché à obtenir des produits à propriétés améliorées, en particulier au niveau de leur liposolubilité et de leur pouvoir filtrant, en venant fixer par greffage un ou plusieurs groupements filtrant benzylidène camphre, benzotriazole ou benzalmalonate sur une s-triazine. Cette technique, décrite dans les demandes de brevet EP 0 507 691 et EP 0 507 692 au nom de la Demanderesse, conduit certes à des composés intéressants mais le caractère liposoluble de ces derniers peut encore apparaître comme insuffisant et, de plus, afin d'obtenir des propriétés filtrantes satisfaisantes avec ce type de produits, il est souvent nécessaire de mettre en oeuvre des quantités relativement importantes de ces filtres, ce qui se traduit par de mauvaises propriétés cosmétiques au niveau des formulations qui les contiennent.

De même, dans les documents EP 0 517 104 et US 4, 724, 137, on a cherché à augmenter le SPF de dérivés de l'acide p-aminobenzoïque en greffant sur une s-triazine trois groupements p-aminobenzoates. On obtient des composés présentant un bon SPF mais dont la liposolubilité est très faible. La formulation de tels composés s'avère difficile et fastidieuse et nécessite l'ajout de substances solubilisantes en quantité parfois importante.

La présente invention vise à résoudre les problèmes ci-dessus en proposant de nouveaux composés, de type s-triazine silanique ou siloxanique, à fonction aminobenzylidène camphre, aminobenzalmalonate, aminobenzoate, ou encore aminobenzotriazole, aminobenzimidazole, aminobenzoxazole, aminobenzo-thiazole, aminosalicylate, aminocinnamate ou/et amino cinnamonitrile, qui présentent des propriétés améliorées, notamment au niveau de leur solubilité dans les corps gras et de leur pouvoir d'absorption intrinsèque vis-à-vis des UV.

Plus précisément encore, il a été trouvé, selon la présente invention, qu'en greffant sur une s-triazine, d'une part, au moins une chaîne siliconée particulière ou un silane particulier et, d'autre part, un ou plusieurs chromophores filtrants, il était possible d'aboutir à de nouveaux composés obviant aux inconvénients des filtres de l'art antérieur, ces nouveaux composés présentant, outre des propriétés filtrantes très élevées, une très bonne solubilité dans les solvants organiques usuels et notamment les corps gras tels que les huiles, ainsi que d'excellentes propriétés cosmétiques, les rendant particulièrement appropriés à une utilisation comme filtres solaires dans des, ou pour la préparation de, compositions cosmétiques destinées à la protection de la peau et/ou des cheveux contre le rayonnement ultraviolet.

Par chromophore filtrant, on entend, au sens de la présente invention, un groupement absorbant les rayonnements UV dans la zone comprise entre 280 nm et 400 nm. Les chromophores filtrants convenant particulièrement bien à la préparation des composés selon l'invention sont les dérivés d'aminobenzylidène camphre, d'aminobenzalmalonate, d'aminobenzoate ou encore d'amino-benzotriazole, d'aminobenzimidazole, d'aminobenzoxazole, d'aminobenzo-thiazole, d'aminosalicylate, d'aminocinnamate ou/et d'amino cinnamonitrile.

La présente invention a ainsi pour premier objet de nouveaux composés qui sont caractérisés par le fait qu'ils répondent à la formule générale (I) suivante : 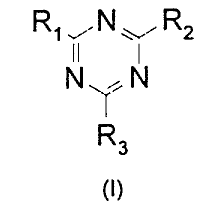 dans laquelle :
- R₁ représente un radical -NH-F,
- R₂ représente un radical -NH-Z-W,
- R₃ est R₁, R₂, un radical alkyle en C₁-C₂₀, linéaire ou ramifié, un radical hydroxyalkyle en C₂-C₂₀, linéaire ou ramifié, un radical alcoxy en C₁-C₂₀, linéaire ou ramifié, le radical NHR₈ ou le radical N(R₈)₂ avec R₈ étant un radical alkyle en C₁-C₂₀, linéaire ou ramifié,
- F représente un chromophore filtrant,
- Z représente un radical divalent assurant la liaison entre -NH- et -W,
- W représente soit un radical siliconé comprenant au moins une unité de formule (1) suivante : 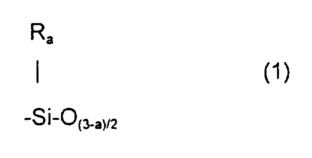 dans laquelle R désigne un groupe hydrocarboné saturé ou insaturé en C₁-C₃₀, un groupe hydrocarboné halogéné en C₁-C₈ ou un groupe triméthylsilyloxy et a est égal à 1 ou 2,
- soit un radical de formule (2) suivante :

   -SiR'₁R'₂R'₃ (2)

   dans laquelle R'₁, R'₂, R'₃, identiques ou différents, sont choisis parmi les radicaux alkyles et alcényles en C₁-C₈, linéaires ou ramifiés.

De manière préférentielle, le chromophore filtrant F est choisi indépendamment parmi les dérivés de benzylidène camphre, de benzalmalonate, de benzoate ou encore de benzotriazole, de benzimidazole, de benzoxazole, de benzothiazole, de salicylate, de cinnamate ou/et de cinnamonitrile. Ce chromophore F est de manière encore plus préférentielle choisi indépendamment parmi les radicaux de formules A à E suivantes : 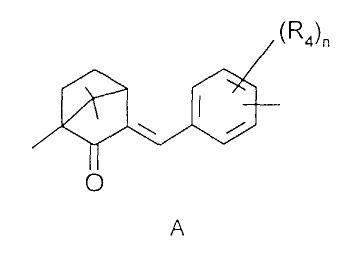 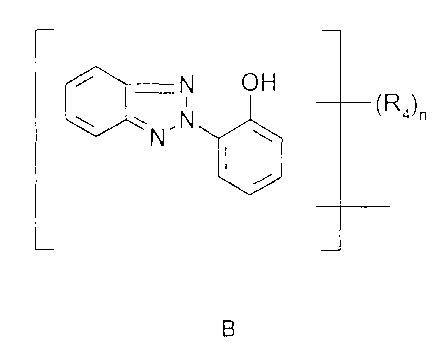 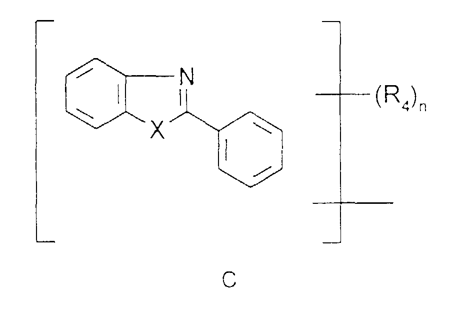 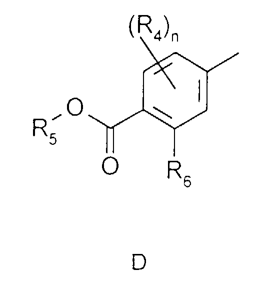 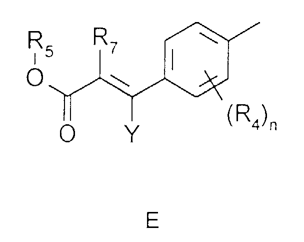 dans lesquelles :
- R₄, identiques ou différents, désignent un radical alkyle en C₁-C₈ linéaire ou ramifié, un radical alcoxy en C₁-C₄, deux R₄ adjacents d'un même noyau aromatique pouvant former ensemble un groupement alkylidène dioxy dans lequel le groupe alkylidène contient 1 ou 2 atomes de carbone,
- n est 0, 1 ou 2,
- R₅ est un radical alkyle en C₁-C₂₀, linéaire ou ramifié,
- R₆ représente un atome d'hydrogène, un radical hydroxyle ou un radical alcoxy en C₁-C_{6,}
- R₇ représente un atome d'hydrogène, COOR₅ ou le radical cyano,
- X est un atome d'oxygène, de soufre ou le groupement N-R₉ avec R₉ étant un atome d'hydrogène ou un radical alkyle en C₁-C₄ linéaire ou ramifié,
- Y représente un atome d'hydrogène ou un radical phényle éventuellement substitué par un radical alkyle ou alcoxy en C₁-C_{4,}

Le radical divalent Z est préférentiellement choisi parmi les radicaux de formules (a) ou (b) suivantes : 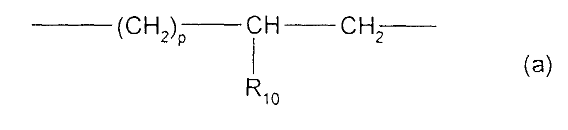 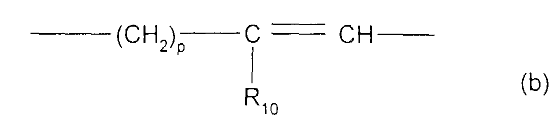 dans lesquelles R₁₀ représente un atome d'hydrogène ou un radical alkyle en C₁-C₈, linéaire ou ramifié, saturé ou insaturé et p est un nombre entier compris entre 0 et 10.

Dans une forme préférée de l'invention, W est un radical siliconé répondant à l'une des trois formules (3) à (5) suivantes : 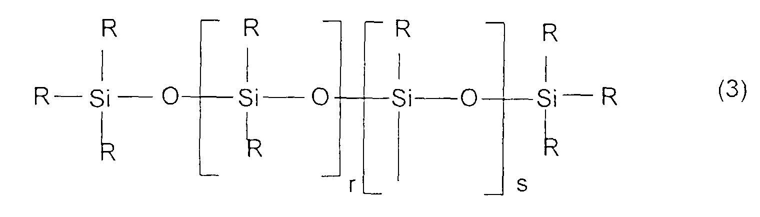 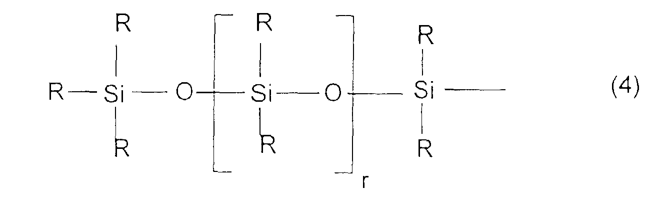 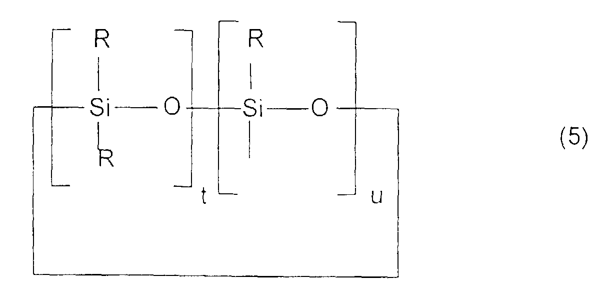 dans lesquelles :
- R, identiques ou différents sont choisis parmi les radicaux alkyles en C₁-C₁₀, linéaires ou ramifiés, saturés ou insaturés, le radical phényle et le radical trifluoro-3,3,3 propyle, au moins 80% en nombre des radicaux R étant le radical méthyle,
- r est un nombre entier choisi entre 0 et 50 inclusivement,
- s est un nombre entier choisi entre 0 et 20 inclusivement,
- u est un nombre entier compris entre 1 et 6 inclus,
- t est un nombre entier entre 0 et 10 inclus,
- t + u est égal ou supérieur à 3.

Les composés de l'invention présentent une excellente liposolubilité et peuvent ainsi être utilisés à de grandes concentrations, ce qui confère aux compositions finales des indices de protection très élevés ; par ailleurs, ils se répartissent uniformément dans les supports cosmétiques classiques contenant au moins une phase grasse ou un solvant organique cosmétiquement acceptable et peuvent être ainsi appliqués sur la peau ou les cheveux pour constituer un film protecteur efficace. De plus, leurs propriétés cosmétiques sont très bonnes : en particulier, ces produits apportent de la douceur.

En outre, les composés de l'invention présentent un excellent pouvoir filtrant intrinsèque à l'égard du rayonnement ultraviolet UV-A et/ou UV-B, selon la structure du produit. En effet, selon la nature des groupements R₁ et R₃, on obtiendra un composé final absorbant plus particulièrement dans l'UV-A ou au contraire plus particulièrement dans l'UV-B. Ainsi, si l'on désire obtenir un composé absorbant plus particulièrement dans l'UV-A, on aura intérêt à greffer sur la triazine un ou plusieurs groupements choisis parmi les dérivés aminobenzylidène camphre, aminobenzoxazole, aminobenzalmalonate ou certains dérivés aminobenzotriazole (radicaux A, C, E et certains radicaux B ci-dessus). Au contraire, si l'on désire obtenir un composé absorbant plus particulièrement dans l'UV-B, on aura intérêt à greffer sur la triazine un ou plusieurs groupements choisis parmi les dérivés aminobenzoates ou certains dérivés aminobenzotriazole (certains radicaux B et radicaux D ci-dessus). Un des avantages considérables de la présente invention est ainsi de proposer des filtres UV absorbant dans l'ensemble du rayonnement UV (à savoir dans la gamme de longueur d'onde allant de 280 nm à 400 nm), ces filtres étant obtenus en greffant sur la même triazine à la fois un groupement présentant une forte absorption dans l'UV-A et un groupement présentant une absorption dans l'UV-B (par exemple un composé pour lequel R₁ est égal à un radical -NH-A et R₃ est égal à un radical -NH-D).

Grâce à la présente invention, il est ainsi possible de disposer d'une composition qui présentera globalement une activité filtrante exceptionnelle dans toute la gamme des UV nocifs (UV-A + UV-B), soit en utilisant au moins un composé de l'invention présentant deux groupements différents, l'un absorbant dans l'UV-A et l'autre absorbant dans l'UV-B, soit en mélangeant des produits de structure différente, c'est-à-dire plus précisément en mélangeant des produits conformes à l'invention présentant une activité purement UV-A avec des produits conformes à l'invention présentant une activité purement UV-B.

Ces nouveaux composés s-triaziniques peuvent ainsi être utilisés comme filtres solaires pour la peau humaine et les cheveux. Ils peuvent aussi être utilisés comme agents protecteurs de la lumière dans l'industrie des plastiques.

Dans les formules (1) à (5) ci-dessus, les radicaux alkyle peuvent être linéaires ou ramifiés et choisis notamment au sein des radicaux méthyle, éthyle, n-propyle, isopropyle, n-butyle, isobutyle, ter.-butyle, n-amyle, isoamyle, néopentyle, n-hexyle, n-heptyle, n-octyle, éthyl-2 hexyle et ter.-octyle. Les radicaux alkyle R préférés selon l'invention sont les radicaux méthyle, éthyle, propyle, n-butyle, n-octyle et éthyl-2 hexyle. Encore plus préférentiellement, les radicaux R sont tous des radicaux méthyle.

Parmi les composés de formule (I) ci-dessus, on préfère mettre en oeuvre ceux pour lesquels le radical W répond à la formule (3) ou à la formule (4), c'est-à-dire ceux pour lesquels le radical siliconé est un radical diorganosiloxanique linéaire.

Parmi les radicaux diorganosiloxaniques linéaires rentrant dans le cadre de la présente invention, on préfère plus particulièrement les dérivés statistiques ou bien définis à blocs présentant au moins l'une, et encore plus préférentiellement l'ensemble, des caractéristiques suivantes :
- R est alkyle et encore plus préférentiellement est méthyle,
- r est compris entre 0 et 3 inclusivement ; s est compris entre 0 et 3 inclusivement.

La présente invention a également pour objet le procédé de préparation des composés de formule (I).

Les composés de formule (I) peuvent être obtenus selon le schéma réactionnel ci-dessous: 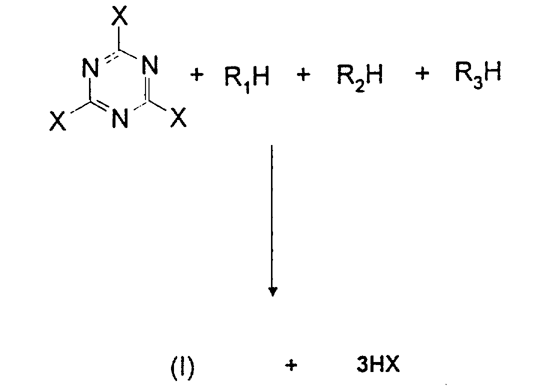 où R₁, R₂ et R₃ répondent aux définitions ci-dessus et X représente un halogène, en particulier le chlore ou le brome.

Les greffages des différents radicaux R₁, R₂ et R₃ ci-dessus sur la s-triazine peuvent se faire indépendamment les uns des autres et dans n'importe quel ordre. De préférence, on greffe en premier lieu sur la s-triazine le ou les groupements correspondant aux radicaux -NH-F puis ensuite le ou groupements -NH-Z-W correspondant aux chaînes aminosiliconées.

Les réactions ci-dessus peuvent être effectuées éventuellement en présence d'un solvant comme par exemple le toluène, le xylène ou encore un mélange acétone/eau.

Les réactions ci-dessus peuvent également éventuellement être réalisées en présence d'une base telle que la soude, les carbonates ou encore une amine.

Les composés R₁H ou R₃H correspondant aux radicaux -NH-F peuvent être préparés selon des méthodes connues.

Ainsi, la préparation des dérivés aminés de benzylidène camphre est décrite dans le document « Haller, Boudin, Annales de Chimie, 9ème série, tome XVII (1922) ». Comme dérivé aminé de benzylidène camphre convenant particulièrement bien à la préparation des composés selon l'invention, on peut citer le 4-amino benzylidène camphre.

La préparation des dérivés aminés de benzalmalonate est par exemple décrite dans GB 1,064,116. Comme dérivés aminés de benzalmalonate convenant particulièrement bien à la préparation des composés de l'invention, on peut citer le 4-amino benzalmalonate de diméthyle, le 4-amino benzalmalonate de diéthyle, le 4-amino benzalmalonate de diisopropyle ou encore le 4-amino benzalmalonate de diisobutyle.

La préparation des dérivés aminés d'acide benzoïque est décrite notamment dans FR2,151,503. Comme dérivés aminés d'acide benzoïque convenant particulièrement bien à la préparation des composés selon l'invention, on peut citer le 4-amino benzoate d'éthyle et le 4-amino benzoate de butyle.

La préparation des dérivés aminés de benzotriazole est décrite par exemple dans FR 1,324,897 ou encore dans l'article de T.Konstantinova et al, Polymer Degradation and Stability, 43, 187 (1994). Comme dérivés aminés de benzotriazole convenant particulièrement bien à la préparation des composés selon l'invention, on peut notamment citer le 2-(2-hydroxy-5-aminophenyl)-5-methoxybenzotriazole décrit dans EP 221,630, le 2-(2-hydroxy-4-aminophenyl)benzotriazole décrit dans US 3,159,646, le 2-(2-hydroxyphenyl)-5-aminobenzotriazole décrit dans US 3,159,646 et GB 1,346,764, le 2-(2-hydroxy-5-methylphenyl)-5-aminobenzotriazole décrit dans US 3,159,646, le 2-(2-hydroxy-5-aminophenyl)benzotriazole décrit dans l'article de J.Belusa et al, Chem.Zvesti, le 2-(2-hydroxy-5-aminophenyl)-5-chorobenzotriazole décrit dans l'article de H.S.Freeman et al, Dyes and Pigments, 20, 171 (1992), le 2-(2-hydroxy-4-aminophenyl)-5-chorobenzotriazole décrit dans l'article de H.S.Freeman et al, Dyes and Pigments, 20, 171 (1992), le 2-(2-hydroxy-3-amino-5-methylphenyl)benzotriazole décrit dans DE 2,128,005 et GB1,346,764, le 2-(2-hydroxy-3-amino-5-t-butylphenyl)benzotriazole décrit dans DE 2,128,005 et GB 1,346,764.

La préparation des dérivés aminés de benzoxazole est notamment décrite dans US 2,334,348. Comme dérivés aminés de benzoxazole convenant particulièrement bien à la préparation des composés de l'invention, on peut citer en particulier le 2-phényl-5-aminobenzoxazole.

La préparation des dérivés aminés de benzothiazole est par exemple décrite dans US 2,334,348. Comme dérivés aminés de benzothiazole convenant particulièrement bien à la préparation des composés de l'invention, on peut citer en particulier le 2-(para-amino phényl)-6-methylbenzothiazole.

La préparation des dérivés aminés d'acide salicylique est notamment décrite dans FR 2,385,685. Comme dérivés aminés d'acide salicylique convenant particulièrement bien à la préparation des composés selon l'invention, on peut citer le 4-amino salicylate d'éthyle ou encore le 4-amino salicylate d'isobutyle ou encore le 5- amino salicylate d'éthyle.

Comme dérivé aminé d'acide cinnamique, on peut par exemple citer le 4-amino cinnamate d'éthyle.

La préparation des dérivés aminés de cyano acrylate est par exemple décrite dans l'article J.Soc.Dyers Colour. (1977), 93, p126-133. Comme dérivés aminés de cyano acrylate convenant particulièrement bien à la préparation des composés de la présente invention, on peut citer l'α-cyano-4-aminocinnamate d'éthyle, l'α-cyano-4-aminocinnamate d'isopropyle ou encore l'α-cyano-4-aminocinnamate d'éthyl-2-hexyle.

Les composés R₂H et R₃H correspondant aux chaînes aminosiliconées (radicaux -NH-Z-W) peuvent également être préparés selon des procédés connus.

La préparation des aminosiloxanes est par exemple décrite dans GB 2 185 984. Comme aminosiloxanes convenant particulièrement bien à la préparation des composés selon l'invention, on peut citer l'aminopropyl heptaméthyl trisiloxane, l'aminoisobutyl heptaméthyl trisiloxane, ou encore les triméthylsilylamo-diméthicone telles que : le produit vendu sous la dénomination commerciale «X₂-8260» par la société DOW CORNING, d'indice d'amine 2,8 meq/gramme ; le produit vendu sous la dénomination commerciale «SLM 55051/3» par la société WACKER, d'indice d'amine 0,47 meq/gramme ; les PDMS diméthylalkyle en C₁₂, telles que le produit vendu sous la dénomination commerciale «SLM 23046/1» par la société WACKER, d'indice d'amine 1,2 meq/gramme ; les polyméthylalkyl (gras) arylalkylsiloxane α, ω triméthylées, telles que le produit vendu sous la dénomination commerciale «SLM 23056/2 » par la société WACKER, d'indice d'amine 1,3 meq/gramme ; les PDMS dont le radical NH₂ est en position α et ω sur un site alkyle telles que les produits vendus sous les dénominations commerciales «TEGOMER A-SI 2120», d'indice d'amine 1,95 meq/gramme et «TEGOMER A-SI 2320», d'indice d'amine 0,86 meq/gramme, par la société GOLDSCHMIDT.

La préparation des aminosiloxanes cycliques est par exemple décrite dans l'article de A.Kopylov, Zh.Obshch. Khim., 54(2), 367-71 (1984).

La préparation des aminosilanes est par exemple décrite dans EP 321 174.

Comme dérivés aminosilanes convenant particulièrement bien à la préparation des composés de la présente invention, on peut citer l'aminopropyl triméthylsilane et l'aminopropyl triéthylsilane.

La présente invention a également pour objet une composition comprenant un composé de formule (I) selon l'invention dans un support approprié. Le support peut être par exemple une composition de matière plastique. Il peut également être approprié pour une application topique. Dans ce cas, la composition selon l'invention est une composition cosmétique qui comprend un support cosmétiquement acceptable.

De préférence, la composition selon l'invention est une composition destinée à protéger une matière sensible au rayonnement ultraviolet, en particulier au rayonnement solaire, comprenant une quantité efficace d'au moins un composé conforme à l'invention. Dans une forme préférée de réalisation de l'invention, cette composition est destinée à protéger la peau et/ou les cheveux.

Les composés de formule (I) sont généralement présents dans la composition de l'invention dans des proportions comprises entre 0,1 % et 20 % en poids, de préférence entre 0.5 % et 10 % en poids, par rapport au poids total de la composition.

Les compositions selon l'invention peuvent bien entendu contenir un ou plusieurs filtres solaires complémentaires actifs dans l'UVA et/ou l'UVB (absorbeurs), autres que les composés conformes à la présente invention, hydrophiles ou lipophiles. Ces filtres complémentaires peuvent être notamment choisis parmi les dérivés cinnamiques, les dérivés salicyliques, les dérivés du camphre, les dérivés de triazine, les dérivés de la benzophénone, les dérivés du dibenzoylméthane, les dérivés de β,β-diphénylacrylate, les dérivés de l'acide p-aminobenzoïque, les polymères filtres et silicones filtres décrits dans la demande WO-93/04665. D'autres exemples de filtres organiques sont donnés dans la demande de brevet EP-A 0 487 404.

Les compositions selon l'invention peuvent également contenir des agents de bronzage et/ou de brunissage artificiels de la peau (agents autobronzants), tels que par exemple de la dihydroxyacétone (DHA).

Les compositions selon l'invention peuvent encore contenir des pigments ou bien encore des nanopigments (taille moyenne des particules primaires : généralement entre 5 nm et 100 nm, de préférence entre 10 nm et 50 nm) d'oxydes métalliques enrobés ou non comme par exemple des nanopigments d'oxyde de titane (amorphe ou cristallisé sous forme rutile et/ou anatase), de fer, de zinc, de zirconium ou de cérium qui sont tous des agents photoprotecteurs UV bien connus en soi. Des agents d'enrobage classiques sont par ailleurs l'alumine et/ou le stéarate d'aluminium. De tels nanopigments d'oxydes métalliques, enrobés ou non enrobés, sont en particulier décrits dans les demande de brevets EP-A- 0 518 772 et EP-A- 0 518 773.

Elle peut contenir les adjuvants cosmétiques habituellement utilisés dans le domaine cosmétique, tels que des corps gras, des solvants organiques, des silicones, des épaississants, des adoucissants, des filtres solaires complémentaires, des agents anti-mousses, des agents hydratants, des parfums, des conservateurs, des tensio-actifs, des charges, des séquestrants, des polymères anioniques, cationiques, non ioniques ou amphotères ou leurs mélanges, des propulseurs, des agents alcalinisants ou acidifiants, des colorants, des pigments ou nanopigments en particulier ceux destinés à assurer un effet photoprotecteur complémentaire par blocage physique du rayonnement ultraviolet, ou tout autre ingrédient habituellement utilisé en cosmétique, en particulier pour la fabrication des compositions antisolaires.

Parmi les solvants organiques, on peut citer les alcools et polyols inférieurs tels que l'éthanol, l'isopropanol, le propylèneglycol, la glycérine et le sorbitol.

Les corps gras peuvent être constitués par une huile ou par une cire ou leurs mélanges, des acides gras, des esters d'acides gras, des alcools gras, la vaseline, la paraffine, la lanoline, la lanoline hydrogénée, la lanoline acétylée. Les huiles peuvent être choisies parmi les huiles animales, végétales, minérales ou de synthèse, et notamment l'huile de palme hydrogénée, l'huile de ricin hydrogénée, l'huile de vaseline, l'huile de paraffine, l'huile de Purcellin, les huiles de silicones, volatiles ou non, et les isoparaffines.

Bien entendu, l'homme de l'art veillera à choisir le ou les éventuels composés complémentaires cités ci-dessus et/ou leurs quantités de manière telle que les propriétés avantageuses attachées intrinsèquement au composé conforme à l'invention ne soient pas, ou substantiellement pas, altérées par la ou les adjonctions envisagées.

La composition cosmétique de l'invention peut être utilisée comme composition protectrice de l'épiderme humain ou des cheveux contre les rayons ultraviolets, comme composition antisolaire ou comme produit de maquillage.

Cette composition peut se présenter en particulier sous forme de lotion, de lotion épaissie, de gel, de crème, de lait, de poudre, de bâtonnet solide et éventuellement être conditionnée en aérosol et se présenter sous forme de mousse ou de spray.

Lorsque la composition cosmétique selon l'invention est plus particulièrement destinée à la protection de l'épiderme humain contre les rayons UV ou comme composition antisolaire, elle peut se présenter sous forme de suspension ou de dispersion dans des solvants ou des corps gras, ou encore sous forme d'émulsion (notamment de type H/E ou E/H, mais de préférence H/E) telle qu'une crème ou un lait, de dispersion vésiculaire, sous forme de pommade, de gel, de bâtonnet solide ou de mousse aérosol. Les émulsions peuvent contenir en outre des agents tensio-actifs anioniques, non ioniques, cationiques ou amphotères.

Lorsque la composition cosmétique selon l'invention est utilisée pour la protection des cheveux, elle peut se présenter sous forme de shampooing, de lotion, de gel ou composition à rincer, à appliquer avant ou après shampooing, avant ou après coloration ou décoloration, avant, pendant ou après permanente ou défrisage, de lotion ou gel coiffant ou traitant, de lotion ou gel pour le brushing ou la mise en plis, de laque pour cheveux, de composition de permanente ou de défrisage, de coloration ou décoloration des cheveux.

Lorsque la composition cosmétique selon l'invention est utilisée comme produit de maquillage des cils, des sourcils, de la peau ou des cheveux, tels que crème de traitement de l'épiderme, fond de teint, bâton de rouge à lèvres, fards à paupière, fards à joues, ligneur encore appelé "eye-liner", mascara, gel colorant, elle peut se présenter sous forme solide ou pâteuse, anhydre ou aqueuse, comme des émulsions huile-dans-eau ou eau-dans-huile, des suspensions ou encore des gels.

L'invention a encore pour objet l'utilisation d'un composé conforme à l'invention dans des, ou pour la fabrication de, compositions destinées à protéger des matières sensibles au rayonnement ultraviolet, en particulier au rayonnement solaire.

L'invention a encore pour objet l'utilisation d'un composé de formule générale (I) conforme à l'invention pour la préparation d'un médicament destiné à prévenir les effets néfastes des rayonnements UV.

L'invention a encore pour objet l'utilisation d'un composé de formule générale (I) conforme à l'invention à titre d'agent filtrant les rayonnements UV, en particulier pour contrôler la couleur de la peau.

L'invention a enfin pour objet un procédé non thérapeutique de protection de la peau et/ou des cheveux contre le rayonnement ultraviolet, en particulier le rayonnement solaire, qui consiste à appliquer sur la peau ou les cheveux une quantité efficace de la composition cosmétique définie ci-dessus, ou d'un composé de formule (I) tel que défini ci-avant.

L'invention a finalement pour objet un procédé non thérapeutique de contrôle de la variation de couleur de la peau due aux rayonnements UV, qui consiste à appliquer sur la peau une quantité efficace de la composition cosmétique définie ci-dessus, ou d'un composé de formule (I) tel que défini ci-avant.

Les exemples qui suivent illustrent l'invention sans toutefois en limiter la portée.

### EXEMPLE 1:

### Préparation du 2-(4'-ylamino benzoate de butyle)-4,6-bis{[1,3,3,3-tetramethyl-1-[(trimethylsilyl)oxy]disiloxany]propyl-3-diylamino}-s-triazine :

### 1ère étape : préparation de la 2-(4'-ylamino benzoate de butyle)-4,6-dichloro-s-triazine:

A une solution de chlorure de cyanuryle (18,4 g, 0,1 mole) dans 100 ml d'acétone/50 ml d'eau, on ajoute à 0-5°C, goutte à goutte, le 4-amino benzoate de butyle (19,32 g, 0,1 mole) en solution dans 200 ml d'acétone, puis 100 ml de bicarbonate de sodium 0,1 N en 1 heure. L'agitation est maintenue 1 heure. Après essorage du précipité, lavage à l'eau et séchage, on obtient la 2-(4'-ylamino benzoate de butyle)-4,6-dichloro-s-triazine (33 g, rendement = 96 %) ayant les caractéristiques suivantes :
- poudre blanche
- Pf :248 °C
- UV (Ethanol à 95%) λₘₐₓ = 294 nm, εₘₐₓ = 28 960

| Analyse élémentaire pour C₁₄ H₁₄ Cl₂ N₄ O₂ : | | | | | |
|---|---|---|---|---|---|
| calculé | C : 49,28 | H : 4,14 | Cl : 20,78 | N : 16,42 | O : 9,38 |
| trouvé | C : 49,32 | H : 4,18 | Cl : 20,94 | N : 16,44 | O : 9,61 |

### 2ème étape : préparation du composé de l'exemple 1

Le dérivé précédent (0,2 g, 5,86x10⁻⁴ mole) et l'amino-1[1,3,3,3-tetramethyl-1-[(trimethylsilyl)oxy]disiloxanyl]-3-propane (0,8 g, 28,7x10⁻⁴ mole) sont chauffés à 90°C pendant 2 heures sous azote. Le mélange réactionnel est chromatographié sur silice (éluant : Heptane/Acétate d'éthyle 70:30). On obtient 0,34 g (Rendement : 70 %) du dérivé de l'exemple 1 sous forme d'une huile incolore :
UV (Ethanol) λₘₐₓ = 300 nm, εₘₐₓ = 40 800

| Analyse élémentaire pour C₃₄ H₇₀ N₆ O₆ Si₆ | | | | |
|---|---|---|---|---|
| calculé | C49,35 | H8,53 | N10,16 | Si20,36 |
| trouvé | C48,95 | H8,39 | N10,05 | Si20,28 |

### EXEMPLE 2:

### Préparation du 2,4-bis(4'-diylamino benzylidene camphre)-6-{[1,3,3,3-tetramethyl-1-[(trimethylsilyl)oxy]disiloxanyl]propyl-3-ylamino}-s-triazine:

### 1ère étape : préparation de la 2,4-bis(4'-diylamino benzylidène camphre)-6-chloro-s-triazine

A une solution de chlorure de cyanuryle (4,61 g, 0,025 mole) dans 50 ml d'acétone on ajoute à 35-40°C, goutte à goutte, le 4-amino benzylidène camphre (12,76 g, 0,05 mole) en solution dans 75 ml d'acétone, puis 50 ml de soude N en 2 heures. L'agitation est maintenue 1,5 heure. Après essorage du précipité, lavage à l'eau et séchage, on obtient la 2,4-bis(4'-diylamino benzylidène camphre)-6-chloro-s-triazine (12,6 g, rendement = 81 %) ayant les caractéristiques suivantes :
- poudre jaune pâle
- Pf : 255 °C
- UV (Ethanol à 95%) λₘₐₓ = 346 nm, εₘₐₓ = 61 900

| Analyse élémentaire pour C₃₇ H₄₀ Cl N₅ O₂ | | | | | |
|---|---|---|---|---|---|
| calculé | C : 71,42 | H : 6,48 | Cl : 5,70 | N : 11,26 | O : 5,14 |
| trouvé | C : 71,43 | H : 6,51 | Cl : 5,66 | N : 11,15 | O : 5,35 |

### 2ème étape : préparation du composé de l'exemple 2:

Le dérivé précédent (0,075g, 1,2x10⁻⁴ mole) et l'amino-1[1,3,3,3-tetramethyl-1-[(trimethylsilyl)oxy]disiloxanyl]-3-propane (0,375 g, 13,4x10⁻⁴ mole) sont chauffés à 90°C pendant 1 heure sous azote. Le mélange réactionnel est chromatographié sur silice (éluant : Heptane/Acétate d'éthyle 70:30). On obtient 0,07 g (Rendement : 70 %) du dérivé de l'exemple 2 ayant les caractéristiques suivantes:
- poudre jaune pâle
- Pf:107°C
- UV (Ethanol ) λₘₐₓ = 354 nm, εₘₐₓ = 66 460

| Analyse élémentaire pour :C₄₇ H₆₈ N₆ O₄ Si₃ | | | | |
|---|---|---|---|---|
| calculé | C : 65.24 | H : 7.92 | N : 9.71 | Si : 9,74 |
| trouvé | C : 64.98 | H : 8.00 | N : 9.49 | Si : 9,46 |

### EXEMPLE 3:

### Préparation du 2,4-bis(4'-diylamino benzoate de butyle)-6-{[1,3,3,3-tetramethyl-1-[(trimethylsilyl)oxy]disiloxanyl]propyl-3-ylamino}-s-triazine:

### 1ère étape : préparation de la 2,4-bis(4'-diylamino benzoate de butyle)-6-chloro-s-triazine:

A une solution de chlorure de cyanuryle (9,2 g, 0,05 mole) dans 100 ml de dioxane, on ajoute goutte à goutte l'aminobenzoate de butyle (9,94g, 0,0515 mole) en solution dans 15 ml de dioxane, puis 3,5 g de carbonate de potassium en solution dans 15 ml d'eau. Le milieu réactionnel est ensuite porté à 40°C et on introduit à nouveau 9,94 g d'aminobenzoate de butyle en solution dans 15 ml de dioxane puis 3,5 g de carbonate de potassium. L'agitation est ensuite maintenue 3 heures à 65°C. Le milieu réactionnel est filtré. Au filtrat, on ajoute 250 ml d'eau. Le précipité formé est essoré, séché et recristallisé dans 250 ml de toluène. On obtient 16.5g (Rendement : 66%) du dérivé attendu sous forme d'une poudre blanche.

### 2ème étape : préparation du composé de l'exemple 3 :

Le dérivé précédent (13,7 g, 0,0275 mole) et l'amino-1[1,3,3,3-tetramethyl-1-[(trimethylsilyl)oxy]disiloxanyl]-3-propane (19,53g, 0,075 mole) dans 20 ml de touène sont portés au reflux pendant 3 heures. Après concentration, le milieu réactionnel est chromatographié sur silice (éluant : heptane/acétate d'éthyle 60:40). On obtient 16g (Rendement : 78%) du dérivé de l'exemple 3 ayant les caratéristiques suivantes :
- poudre blanche
- Pf: 110°C
- UV (Ethanol à 95%) λₘₐₓ = 310 nm, εₘₐₓ = 75 510

| Analyse élémentaire pour C₃₅ H₅₆ N₆ O₆ Si₃ | | | | |
|---|---|---|---|---|
| calculé | C : 56.72 | H : 7.62 | N : 11.34 | Si : 11.37 |
| trouvé | C : 56.42 | H : 7.65 | N : 11,38 | Si : 11.70 |

### EXEMPLE 4:

### Préparation du 2-(2-benzotriazol-2-yl-4-methyl-6-ylamino-phenol)-4,6-bis{[1,3,3,3-tetramethyl-1-[(trimethylsilyl)oxy]disiloxanyl]propyl-3-diylamino}-s-triazine :

### Préparation du 2-(2-Benzotriazol-2-yl-4-methyl-6-ylamino-phenol)-4,6-dichloro-s-triazine:

A une solution de chlorure de cyanuryle (1,46 g, 0,0079 mole) dans 20 ml d'acétone on ajoute 10 ml d'eau puis à 0-5°C, goutte à goutte, le 2-Benzotriazol-2-yl-4-methyl-6-amino-phenol (1,9 g, 0,0079 mole) en solution dans 100 ml de dioxane, puis 0,66g (0,0079mole) de bicarbonate de sodium en solution dans 20 ml d'eau. L'agitation est maintenue 2 heures.; on ajoute 250 ml d'eau. Après essorage du précipité, lavage à l'eau et séchage, on obtient le 2-(2-Benzotriazol-2-yl-4-methyl-6-ylamino-phenol)-4,6-dichloro-s-triazine (3 g, rendement : 100%) ayant les caractéristiques suivantes :
- poudre jaune pâle
- Pf: 221-222 °C

### 2ème étape : préparation du composé de l'exemple 4

Le dérivé précédent (1,55 g, 0,004 mole) et l'amino-1[1,3,3,3-tetramethyl-1-[(trimethylsilyl)oxy]disiloxanyl]-3-propane (6 g, 0,021 mole) sont chauffés à 90°C pendant 2 heures sous azote. Le mélange réactionnel est chromatographié sur silice (éluant : Heptane/dichlorométhane 50:50). On obtient 2,8g (Rendement : 80 %) du dérivé de l'exemple 4 ayant les caractéristiques suivantes :
- poudre jaune pâle
- Pf: 101-102 °C
UV (Ethanol) λₘₐₓ = 324 nm, εₘₐₓ = 22 000

| Analyse élémentaire pour C₃₆ H₆₇ N₉ O₅ Si₆ | | | | |
|---|---|---|---|---|
| calculé | C49,45 | H7.72 | N14.41 | Si19.27 |
| trouvé | C49.52 | H7.71 | N14.21 | Si19.23 |

### EXEMPLE 5:

### Préparation du 2,4-bis(4'-diylamino benzalmalonate de diisobutyle)-6{[1,1,3,3-tétraméthyl-1-[(triméthylsilyl)oxy]disiloxanyl]propyl-3-ylamino}-s-triazine

### Préparation du 2,4-dichloro-6-{[1,1,3,3-tétraméthyl-1-[(triméthylsilyl)oxy]disiloxanyl] propyl-3-ylamino}-s-triazine

A une solution de chlorure de cyanuryle (1,84 g, 0,01 mole) dans 20 ml d'acétone on ajoute à 0°C, goutte à goutte, l'amino-1{[1,1,3,3-tétraméthyl-1-[(triméthylsilyl)oxy]disiloxanyl]propane (2,79 g, 0,01 mole) et une solution de bicarbonate de sodium (0,84g ; 0,01 mole) dans 12 ml d'eau de telle sorte que le pH se situe entre 3 et 5. En fin d'introduction, le pH est 3. L'agitation est maintenue 1 heure à 10°C, puis laissé à température du labo. Le milieu réactionnel est filtré. Le précipité formé est filtré, lavé à l'eau, essoré et séché ; on obtient 3,4 g du produit attendu sous forme de poudre blanche (rendement : 80% et Pf: 59 °C).

### 2ème étape : préparation du composé de l'exemple 6

Le dérivé précédent (1,1 g, 0,0023 mole) et le para-aminobenzalmalonate de diisobutyle (3,19 g ; 0,01 mole) sont chauffés à 90-100°C pendant 1 heure 30 minutes dans 30 ml de toluène. Après concentration, le mélange réactionnel est chromatographié sur silice (éluant: dichlorométhane/méthanol 98:2). On obtient 1,5g (Rendement : 65 %) du dérivé de l'exemple 6 ayant les caractéristiques suivantes :
- cire transparente
- UV (Ethanol) λₘₐₓ = 356,5 nm, εₘₐₓ = 65.650

| Analyse élémentaire pour C₄₉ H₇₆ N₆ O₁₀ Si₃ | | | | |
|---|---|---|---|---|
| calculé | C59,24 | H7.71 | N8,46 | Si8,48 |
| trouvé | C59,13 | H7.70 | N8,34 | Si8,50 |

### EXEMPLE 6:

On donne ici un exemple concret d'une composition cosmétique conforme à l'invention, à savoir une émulsion H/E antisolaire:
- composé de l'exemple 3 5 %
- mélange d'alcool cétylstéarylique et d'alcool cétylstéarylique oxyéthyléné (33 OE) 80/20 vendu sous la dénomination commerciale « DEHSCONET 390 » par TENSIA 7 %
- mélange de mono et distéarate de glycérol vendu sous la dénomination commerciale « CERASYNTH SD » par la société ISP 2 %
- alcool cétylique 1,5 %
- polydiméthylsiloxane vendu sous la dénomination commerciale « DC200 Fluid » par la société DOW CORNING 1,5 %
- benzoate d'alcools en C₁₂-C₁₅ vendu sous la dénomination commerciale « FINSOLV TN » par la société FINETEX 15 %
- glycérine 20 %
- conservateurs qs
- eau déminéralisée qsp 100 %

Cette émulsion H/E solaire est préparée selon les techniques classiques de préparation d'émulsions en dissolvant le filtre dans la phase grasse contenant les émulsionnants, en chauffant cette phase grasse vers 70-80°C et en ajoutant, sous vive agitation, l'eau chauffée à la même température. On maintient l'agitation pendant 10 à 15 minutes, puis on laisse refroidir sous agitation modérée, et vers 40°C on ajoute enfin les conservateurs.

On obtient ainsi une crème antisolaire particulièrement efficace contre les UV B.

## Revendications

1. Composé de formule (I) suivante : 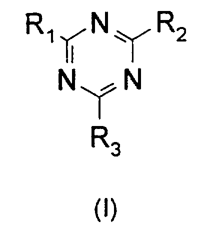 dans laquelle :
- R₁ représente un radical -NH-F,
- R₂ représente un radical -NH-Z-W,
- R₃ est R₁, R₂, un radical alkyle en C₁-C₂₀, linéaire ou ramifié, un radical hydroxyalkyle en C₂-C₂₀, linéaire ou ramifié, un radical alcoxy en C₁-C₂₀, linéaire ou ramifié, le radical NHR₈ ou le radical N(R₈)₂ avec R₈ étant un radical alkyle en C₁-C₂₀, linéaire ou ramifié,
- F représente un chromophore filtrant,
- Z représente un radical divalent assurant la liaison entre -NH- et -W,
- W représente soit un radical silicone comprenant au moins une unité de formule (1) suivante : 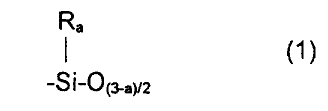 dans laquelle R désigne un groupe hydrocarboné saturé ou insaturé en C₁-C₃₀, un groupe hydrocarboné halogéné en C₁-C₈ ou un groupe triméthylsilyloxy et a est égal à 1 ou 2,
- soit un radical de formule (2) suivante :
-SiR'₁R'₂R'₃ (2)
dans laquelle R'₁, R'₂, R'₃, identiques ou différents sont choisis parmi les radicaux alkyles et alcényles en C₁-C₈, linéaires ou ramifiés.

2. Composé selon la revendication 1, caractérisé par le fait que le chromophore filtrant F est choisi indépendamment parmi les dérivés de benzylidène camphre, de benzalmalonate, de benzoate ou encore de benzotriazole, de benzimidazole, de benzoxazole, de benzothiazole, de salicylate, de cinnamate ou/et de cinnamonitrile.

3. Composé selon la revendication 1 ou 2, caractérisé par le fait que le chromophore F est choisi indépendamment parmi les radicaux de formules A à E suivantes : 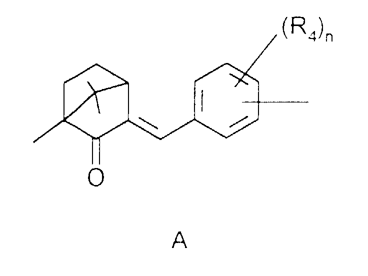 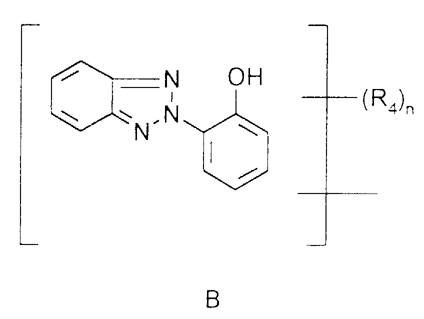 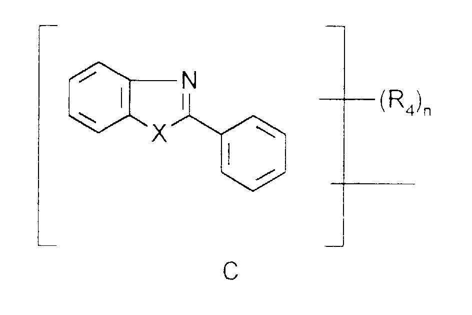 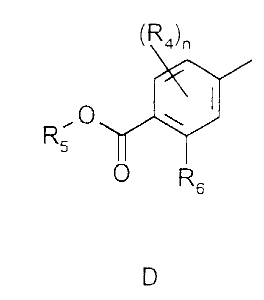 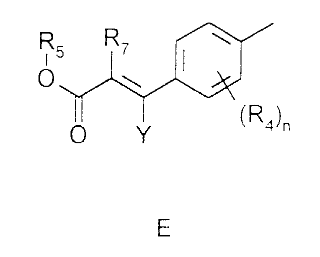 dans lesquelles :
- R₄, identiques ou différents, désignent un radical alkyle en C₁-C₈ linéaire ou ramifié, un radical alcoxy en C₁-C₄, deux R₄ adjacents d'un même noyau aromatique pouvant former ensemble un groupement alkylidène dioxy dans lequel le groupe alkylidène contient 1 ou 2 atomes de carbone,
- n est 0, 1 ou 2,
- R₅ est un radical alkyle en C₁-C₂₀, linéaire ou ramifié,
- R₆ représente un atome d'hydrogène, un radical hydroxyle ou un radical alcoxy en C₁-C₆,
- R₇ représente un atome d'hydrogène, COOR₅ ou le radical cyano,
- X est un atome d'oxygène, de soufre ou le groupement N-R₉ avec R₉ étant un atome d'hydrogène ou un radical alkyle en C₁-C₄ linéaire ou ramifié,
- Y représente un atome d'hydrogène ou un radical phényle éventuellement substitué par un radical alkyle ou alcoxy en C₁-C₄,

4. Composé selon l'une quelconque des revendications précédentes. caractérisé par le fait que ledit radical divalent Z est choisi parmi les radicaux de formules (a) ou (b) suivantes : 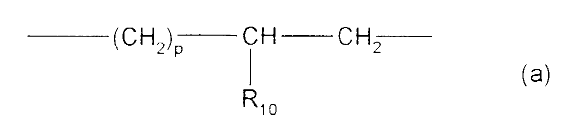 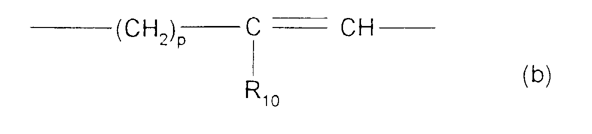 dans lesquelles R₁₀ représente un atome d'hydrogène ou un radical alkyle en C₁-C₈, linéaire ou ramifié, saturé ou insaturé et p est un nombre entier compris entre 0 et 10.

5. Composé selon la revendication 4, caractérisé par le fait que W est un radical siliconé répondant à l'une des trois formules (3) à (5) suivantes : 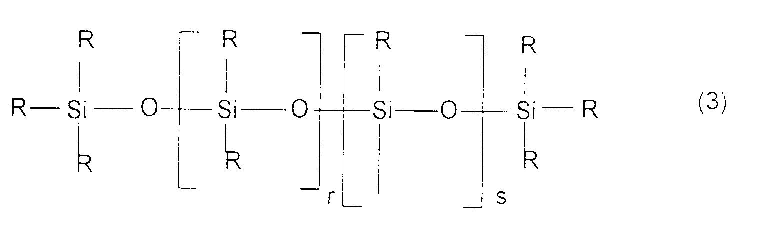 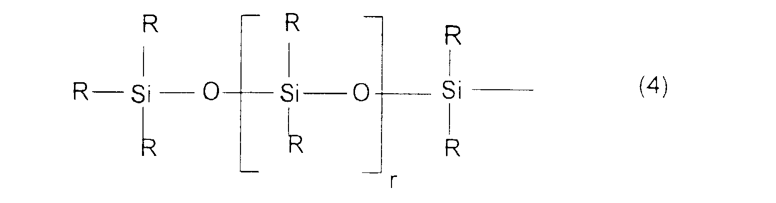 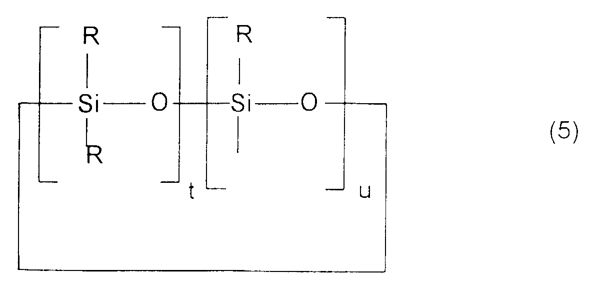 dans lesquelles :
- R, identiques ou différents sont choisis parmi les radicaux alkyles en C₁-C₁₀, linéaires ou ramifiés, saturés ou insaturés. le radical phényle et le radical trifluoro-3,3,3 propyle, au moins 80% en nombre des radicaux R étant le radical méthyle.
- r est un nombre entier choisi entre 0 et 50 inclusivement.
- s est un nombre entier choisi entre 0 et 20 inclusivement,
- u est un nombre entier compris entre 1 et 6 inclus,
- t est un nombre entier entre 0 et 10 inclus,
- t + u est égal ou supérieur à 3.

6. Composé selon la revendication 5, caractérisé par le fait que W répond à la formule (4) ou à la formule (5).

7. Composé selon l'une quelconque des revendications précédentes, caractérisé par le fait qu'il est choisi parmi la 2-(4'-ylamino benzoate de butyle)-4,6-bis{[1,3,3,3-tetramethyl-1-[(trimethylsilyl)oxy]disiloxanyl]propyl-3-diylamino}-s-triazine. 2,4-bis(4'-diylamino benzylidene camphre)-6-{[1,3,3,3-tetramethyl-1-[(trimethylsilyl)oxy]disiloxanyl]propyl-3-ylamino}-s-triazine, la 2,4-bis(4'-diylamino benzoate de butyle)-6-{[1,3,3,3-tetramethyl-1-[(trimethylsilyl)oxy]disiloxanyl]-propyl-3-ylamino}-s-triazine et la 2-(2-benzotriazol-2-yl-4-methyl-6-ylaminophenol)-4,6-bis{[1,3,3,3-tetramethyl-1-[(trimethylsilyl)oxy]disiloxanyl]propyl-3-diylamino}-s-triazine.

8. Procédé de préparation d'un composé tel que défini à l'une quelconque des revendications 1 à 7, caractérisé par le fait qu'il comprend le schéma réactionnel suivant: 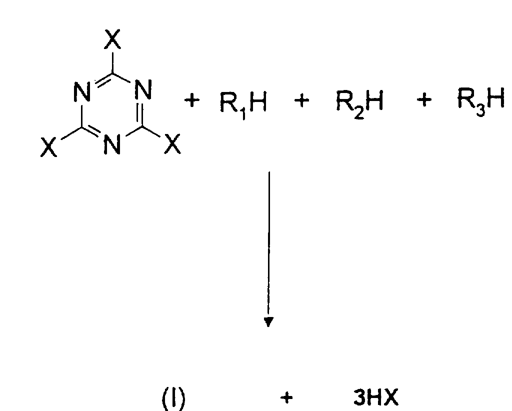 où R₁, R₂ et R₃ répondent aux définitions de la revendication 1 et X représente un halogène, en particulier le chlore ou le brome.

9. Composition cosmétique destinée à protéger la peau et/ou les cheveux du rayonnement UV comprenant dans un support cosmétiquement acceptable au moins un composé de formule (I) tel que défini à l'une quelconque des revendications 1 à 7.

10. Composition de matière plastique, comprenant au moins un composé de formule (I) tel que défini à l'une quelconque des revendications 1 à 7.

11. Composition selon la revendication 9, caractérisée par le fait que le composé de formule (I) tel que défini à l'une quelconque des revendications 1 à 7 est présent dans la composition à une teneur allant de 0,1 à 20 % en poids, par rapport au poids total de la composition, de préférence allant de 0,5 % à 10 % en poids. par rapport au poids total de la composition.

12. Utilisation d'au moins un composé de formule (I) tel que défini à l'une quelconque des revendications 1 à 7 pour la fabrication de compositions destinées à protéger des matières sensibles au rayonnement ultraviolet, en particulier au rayonnement solaire.

13. Utilisation d'au moins un composé de formule (I) tel que défini à l'une quelconque des revendications 1 à 7 pour la préparation d'un médicament destiné à prévenir les effets néfastes des rayonnements UV.

14. Utilisation d'un composé de formule générale (I) tel que défini à l'une quelconque des revendications 1 à 7 dans la fabrication d'une composition cosmétique à titre d'agent de protection de la peau et/ou des cheveux contre le rayonnement ultraviolet, en particulier le rayonnement solaire.

15. Utilisation d'un composé de formule générale (I) tel que défini à l'une quelconque des revendications 1 à 7 dans la fabrication d'une composition cosmétique à titre d'agent de protection de la variation de la couleur de la peau due aux rayonnements UV.

## Patentansprüche

1. Verbindungen der folgenden Formel (I): 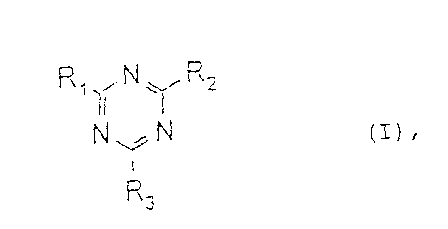 worin bedeuten:
- R₁ eine Gruppe -NH-F,
- R₂ eine Gruppe -NH-Z-W,
- R₃ eine Gruppe R₁ oder R₂, eine geradkettige oder verzweigte C₁₋₂₀-Alkylgruppe, eine geradkettige oder verzweigte C₂₋₂₀-Hydroxyalkylgruppe, eine geradkettige oder verzweigte C₁₋₂₀-Alkoxygruppe, eine Gruppe NHR₈ oder eine Gruppe N(R₈)₂, wobei R₈ eine geradkettige oder verzweigte C₁₋₂₀-Alkylgruppe ist,
- F ein Filterchromophor,
- Z eine zweiwertige Gruppe, die die Bindung von -NH- und -W ermöglicht,
- W entweder eine Silicongruppe, die mindestens eine Einheit der folgenden Formel (1) enthält: 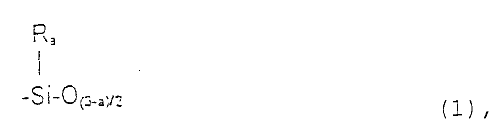 worin R eine gesättigte oder ungesättigte Kohlenwasserstoffgruppe mit 1 bis 30 Kohlenstoffatomen, eine halogenierte Kohlenwasserstoffgruppe mit 1 bis 8 Kohlenstoffatomen oder eine Trimethylsilyloxygruppe bedeutet und a 1 oder 2 ist,
oder eine Gruppe der folgenden Formel (2):
-SiR'₁R'₂R'₃ (2),
worin R'₁, R'₂ und R'₃, die identisch oder voreinander verschieden sind, unter den geradkettigen oder verzweigten Alkyl- oder Alkenylgruppen mit 1 bis 8 Kohlenstoffatomen ausgewählt sind.

2. Verbindung nach Anspruch 1, dadurch gekennzeichnet, daß das Filterchromophor F unter den Benzylidencampherderivaten, Benzalmalonatderivaten, Benzoatderivaten, Benzotriazolderivaten, Benzimidazolderivaten, Benzoxazolderivaten, Benzothiazolderivaten, Salicylatderivaten, Cinnamatderivaten und/oder Cinnamonitrilderivaten ausgewählt ist.

3. Verbindung nach Anspruch 1 oder 2, dadurch gekennzeichnet, daß das Filterchromophor F unter den Gruppen der folgenden Formeln A bis E ausgewählt ist: 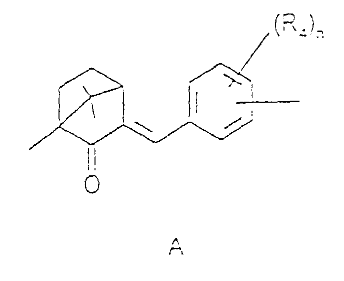 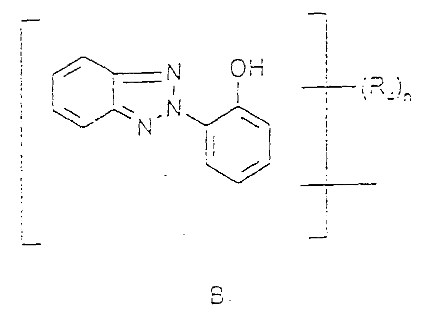 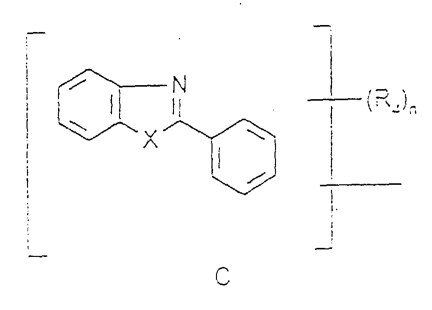 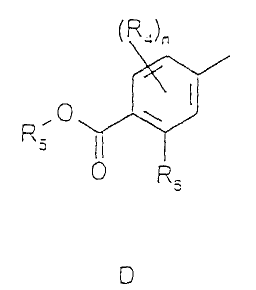 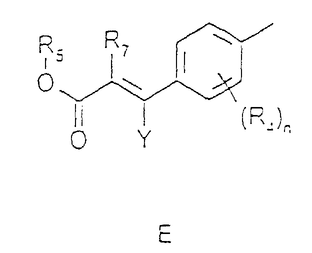 worin bedeuten:
- die Gruppen R₄, die identisch oder voneinander verschieden sind, eine geradkettige oder verzweigte Alkylgruppe mit 1 bis 8 Kohlenstoffatomen oder eine Alkoxygruppe mit 1 bis 4 Kohlenstoffatomen, wobei zwei Gruppen R₄ an einem aromatischen Ring auch gemeinsam eine Alkylidenoxygruppe bilden können, worin die Alkylidengruppe 1 oder 2 Kohlenstoffatome aufweist,
- n 0, 1 oder 2,
- R₅ eine geradkettige oder verzweigte C₁₋₂₀-Alkylgruppe,
- R₆ Wasserstoff, Hydroxy oder C₁₋₆-Alkoxy,
- R₇ Wasserstoff, COOR₅ oder Cyano,
- X Sauerstoff, Schwefel oder eine Gruppe N-R₉, wobei R₉ Wasserstoff oder eine geradkettige oder verzweigte C₁₋₄-Alkylgruppe bedeutet,
- Y Wasserstoff oder eine Phenylgruppe, die gegebenenfalls mit einer Alkylgruppe oder einer Alkoxygruppe mit 1 bis 4 Kohlenstoffatomen substituiert ist.

4. Verbindung nach einem der vorhergehenden Ansprüche, dadurch gekennzeichnet, daß die zweiwertige Gruppe Z unter den Gruppen der folgenden Formeln (a) oder (b) ausgewählt ist: 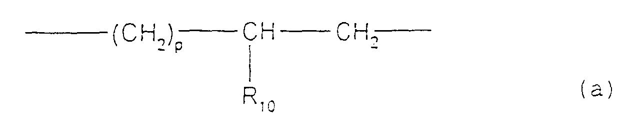 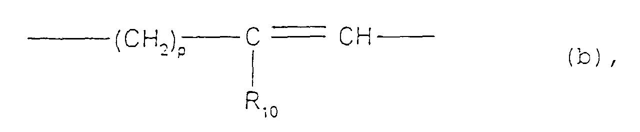 worin R₁₀ Wasserstoff oder eine geradkettige oder verzweigte, gesättigte oder ungesättigte C₁₋₈-Alkylgruppe und p Null oder eine ganze Zahl im Bereich von 1 bis 10 bedeutet.

5. Verbindung nach Anspruch 4, dadurch gekennzeichnet, daß die Gruppe W eine Silicongruppe ist, die einer der folgenden Formeln (3) bis (5) entspricht: 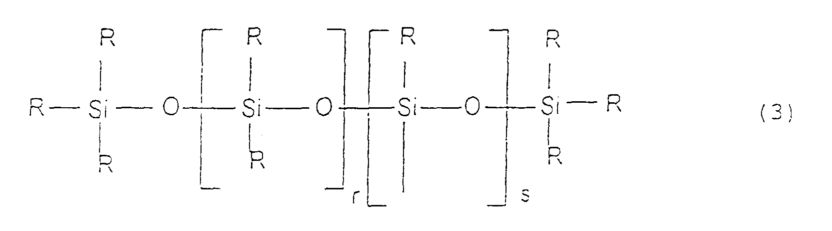 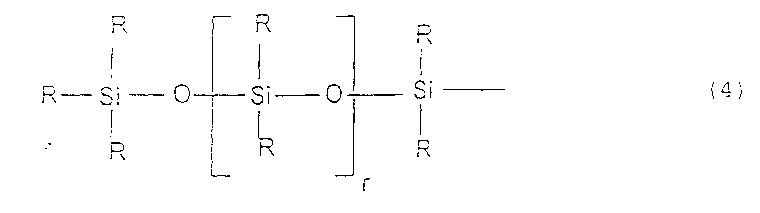 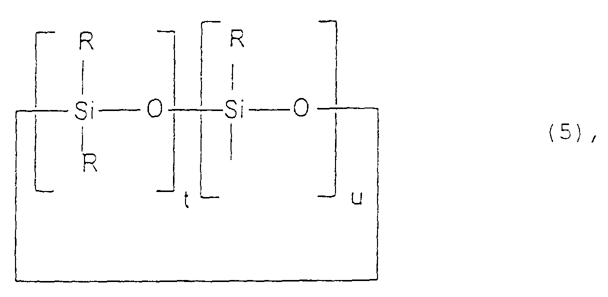 wobei in den Formeln:
- die Gruppen R, die identisch oder voneinander verschieden sind, unter den geradkettigen oder verzweigten, gesättigten oder ungesättigten C₁₋₁₀~Alkylgruppen, Phenyl und 3,3,3-Trifluorpropyl ausgewählt sind, wobei mindestens 80 % der Anzahl der Gruppen R Methyl bedeutet,
- r Null oder eine ganze Zahl im Bereich von 1 bis 50 bedeutet,
- s Null oder eine ganze Zahl im Bereich von 1 bis 20 bedeutet,
- u eine ganze Zahl im Bereich von 1 bis 6 bedeutet,
- t Null oder eine ganze Zahl im Bereich von 1 bis 10 bedeutet, und
- t + u größer oder gleich 3 ist.

6. Verbindung nach Anspruch 5, dadurch gekennzeichnet, daß W der Formel (4) oder der Formel (5) entspricht.

7. Verbindung nach einem der vorhergehenden Ansprüche, dadurch gekennzeichnet, das sie ausgewählt ist unter: 2-(4'-Ylamino-butylbenzoat)-4,6-bis{[1,3,3,3-tetramethyl-1-[(trimethylsilyl)oxy]disiloxanyl]propyl-3-diylamino}-s-triazin, 2,4-Bis-(4'-diylamino-benzylidencampher)-6-{[1,3,3,3-tetramethyl-1-[(trimethylsilyl)oxy]-disiloxanyl]propyl-3-ylamino}-s-triazin, 2,4-Bis-(4'-diylamino-butylbenzoat)-6-{[1,3,3,3-tetramethyl-1-[(trimethylsilyl)-oxy]disiloxanyl]propyl-3-ylamino}-s-triazin und 2-(2-Benzotriazol-2-yl-4-methyl-6-ylaminophenol)-4,6-bis{[1,3,3,3-tetramethyl-1-[(trimethylsilyl)oxy]disiloxanyl]propyl-3-diylamino}-s-triazin.

8. Verfahren zur Herstellung einer Verbindung nach einem der Ansprüche 1 bis 7, gekennzeichnet durch folgendes Reaktionsschema: 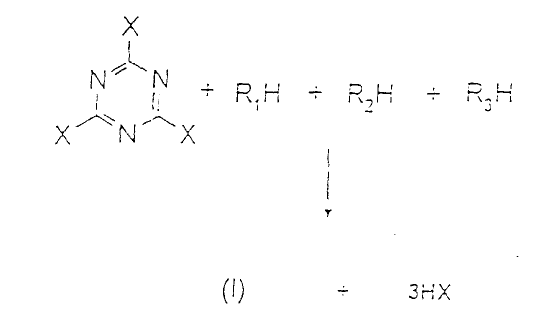 worin R₁, R₂ und R₃ die in Anspruch 1 angegebenen Bedeutungen aufweisen und X Halogen, insbesondere Chlor oder Brom bedeutet.

9. Kosmetische Zusammensetzung zum Schutz der Haut und/oder der Haare gegen UV-Strahlung, die in einem kosmetisch akzeptablen Träger mindestens eine verbindung der Formel (I) nach einem der Ansprüche 1 bis 7 enthält.

10. Zusammensetzung aus Kunststoffmaterial, die mindestens eine Verbindung der Formel (I) nach einem der Ansprüche 1 bis 7 enthält.

11. Zusammensetzung nach Anspruch 9, dadurch gekennzeichnet, daß die Verbindung der Formel (I) nach einem der Ansprüche bis 7 in der Zusammensetzung in einem Mengenanteil von 0,1 bis 20 Gew.-%, bezogen auf das Gesamtgewicht der Zusammensetzung, und vorzugsweise in einem Mengenanteil von 0,5 bis 10 Gew.-%, bezogen auf das Gesamtgewicht der Zusammensetzung, vorliegt.

12. Verwendung mindestens einer Verbindung der Formel (I) nach einem der Ansprüche 1 bis 7 zur Herstellung von Zusammensetzungen, die Materialien schützen sollen, die gegenüber UV-Strahlung und insbesondere gegenüber Sonnenlicht empfindlich sind.

13. Verwendung mindestens einer Verbindung der Formel (I) nach einem der Ansprüche 1 bis 7 zur Herstellung von Arzneimitteln, die den schädlichen Wirkungen der UV-Strahlung vorbeugen sollen.

14. Verwendung einer Verbindung der allgemeinen Formel (I) nach einem der Ansprüche 1 bis 7 zur Herstellung von kosmetischen Zusammensetzungen als Mittel zum Schutz der Haut und/oder der Haare gegen UV-Strahlung und insbesondere gegen Sonnenlicht.

15. Verwendung einer Verbindung der allgemeinen Formel (I) nach einem der Ansprüche 1 bis 7 zur Herstellung von kosmetischen Zusammensetzungen als Mittel zum Schutz gegen die durch die UV-Strahlung hervorgerufene Veränderung der Hautfarbe.

## Claims

1. Compound of following formula (I): 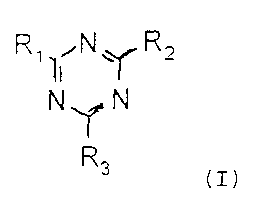 in which:
- R₁ represents an -NH-F radical,
- R₂ represents an -NH-Z-W radical,
- R₃ is R₁, R₂, a linear or branched C₁-C₂₀ alkyl radical, a linear or branched C₂-C₂₀ hydroxyalkyl radical, a linear or branched C₁-C₂₀ alkoxy radical, the NHR₈ radical or the N(R₈)₂ radical, with R₈ being a linear or branched C₁-C₂₀ alkyl radical,
- F represents a screening chromophore,
- Z represents a divalent radical providing the connection between -NH- and -W,
- W represents either a silicone radical comprising at least one unit of following formula (1): 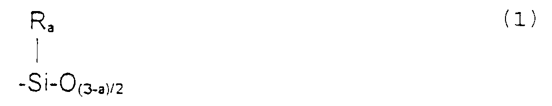 in which R denotes a saturated or unsaturated C₁-C₃₀ hydrocarbon group, a halogenated C₁-C₈ hydrocarbon group or a trimethylsilyloxy group and a is 1 or 2,
- or a radical of following formula (2):
-SiR'₁R'₂R'₃ (2)
in which R'₁, R'₂ and R'₃, which are identical or different, are chosen from linear or branched C₁-C₈ alkyl and alkenyl radicals.

2. Compound according to Claim 1, characterized in that the screening chromophore F is chosen independently from derivatives of benzylidenecamphor, of benzalmalonate, of benzoate or alternatively of benzotriazole, of benzimidazole, of benzoxazole, of benzothiazole, of salicylate, of cinnamate and/or of cinnamonitrile.

3. Compound according to Claim 1 or 2, characterized in that the chromophore F is chosen independently from the radicals of following formulae A to E: 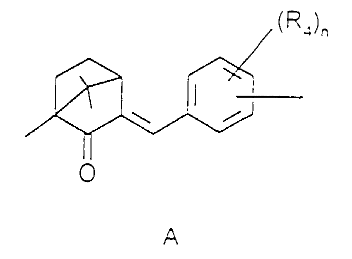 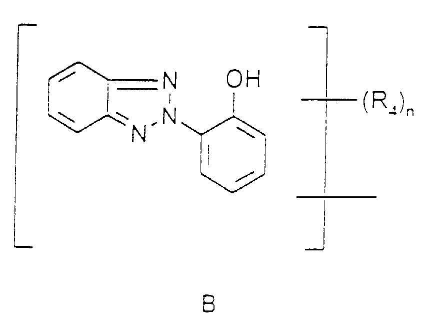 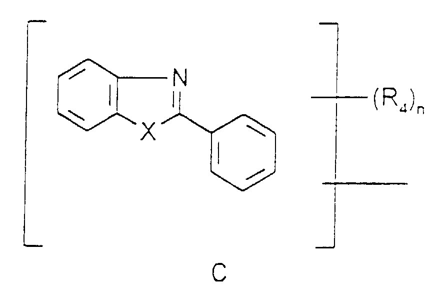 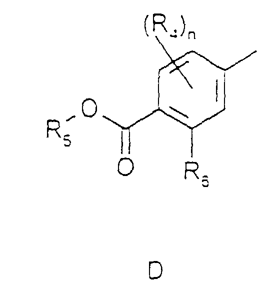 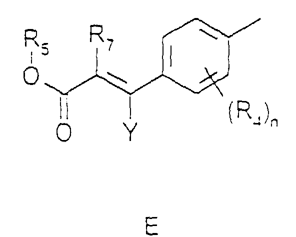 in which:
- R₄, which are identical or different, denote a linear or branched C₁-C₈ alkyl radical or a C₁-C₄ alkoxy radical, it being possible for two adjacent R₄ groups on the same aromatic nucleus together to form an alkylidenedioxy group, in which the alkylidene group contains 1 or 2 carbon atoms,
- n is 0, 1 or 2,
- R₅ is a linear or branched C₁-C₂₀ alkyl radical,
- R₆ represents a hydrogen atom, a hydroxyl radical or a C₁-C₆ alkoxy radical,
- R₇ represents a hydrogen atom, COOR₅ or the cyano radical,
- X is an oxygen or sulphur atom or the N-R₉ group, with R₉ being a hydrogen atom or a linear or branched C₁-C₄ alkyl radical,
- Y represents a hydrogen atom or a phenyl radical optionally substituted by a C₁-C₄ alkyl or alkoxy radical.

4. Compound according to any one of the preceding claims, characterized in that the said divalent radical Z is chosen from the radicals of following formulae (a) or (b): 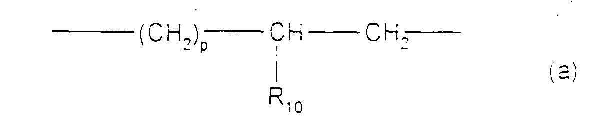 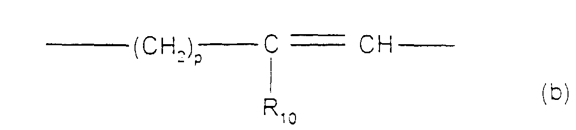 in which R₁₀ represents a hydrogen atom or a saturated or unsaturated, linear or branched C₁-C₈ alkyl radical and p is an integer between 0 and 10.

5. Compound according to Claim 4, characterized in that W is a silicone radical corresponding to one of the three following formulae (3) to (5): 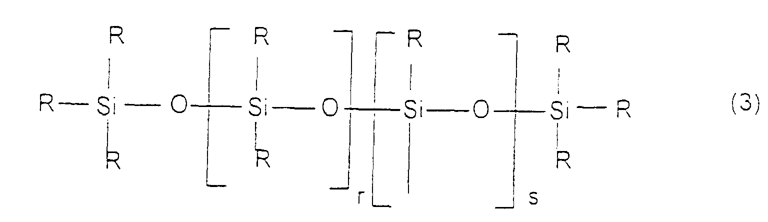 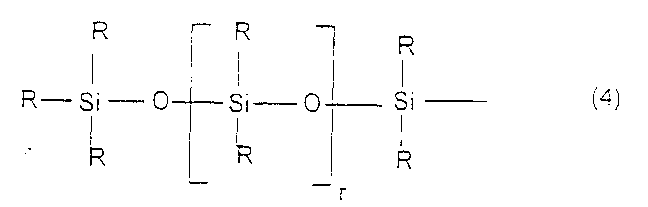 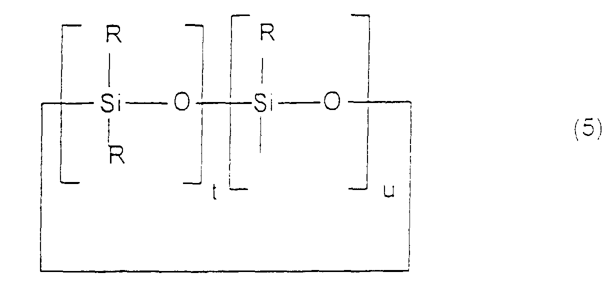 in which:
- R, which are identical or different, are chosen from saturated or unsaturated, linear or branched C₁-C₁₀ alkyl radicals, the phenyl radical and the 3,3,3-trifluoropropyl radical, at least 80% by number of the R radicals being the methyl radical,
- r is an integer chosen between 0 and 50 inclusive,
- s is an integer chosen between 0 and 20 inclusive,
- u is an integer of between 1 and 6 inclusive,
- t is an integer between 0 and 10 inclusive,
- t + u is equal to or greater than 3.

6. Compound according to Claim 5, characterized in that W corresponds to the formula (4) or to the formula (5).

7. Compound according to any one of the preceding claims, characterized in that it is chosen from 2-(butyl 4'-ylaminobenzoate)-4,6-bis{[1,3,3,3-tetramethyl-1-[(trimethylsilyl)oxy]disiloxanyl]propyl-3-ylamino}-s-triazine, 2,4-bis(4'-ylaminobenzylidenecamphor)-6-{[1,3,3,3-tetramethyl-1-[(trimethylsilyl)oxy]disiloxanyl]propyl-3-yl-amino}-s-triazine, 2,4-bis(butyl4'-ylaminobenzoate)-6{[1,3,3,3-tetramethyl-1-[(trimethylsilyl)oxy]disiloxanyl]propyl-3-ylamino}-s-triazine and 2-(2-benzotriazol-2-yl-4-methyl-6-ylaminophenol)-4,6-bis({1,3,3,3-tetramethyl-1-[(trimethylsilyl)oxy]disiloxanyl]propyl-3-ylamino}-s-triazine.

8. Process for the preparation of a compound as defined in any one of Claims 1 to 7, characterized in that it comprises the following reaction scheme: 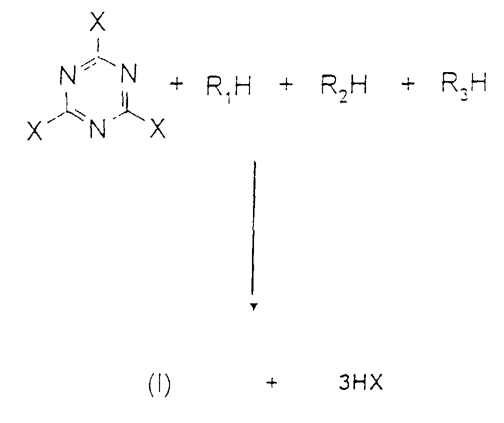 where R₁, R₂ and R₃ correspond to the definitions of Claim 1 and X represents a halogen, in particular chlorine or bromine.

9. Cosmetic composition intended to protect the skin and/or the hair from UV radiation comprising, in a cosmetically acceptable vehicle, at least one compound of formula (I) as defined in any one of Claims 1 to 7.

10. Plastic composition, comprising at least one compound of formula (I) as defined in any one of Claims 1 to 7.

11. Composition according to Claim 9, characterized in that the compound of formula (I) as defined in any one of Claims 1 to 7 is present in the composition at a content ranging from 0.1 to 20% by weight, with respect to the total weight of the composition, preferably ranging from 0.5% to 10% by weight, with respect to the total weight of the composition.

12. Use of at least one compound of formula (I) as defined in any one of Claims 1 to 7 for the manufacture of compositions intended to protect materials sensitive to ultraviolet radiation, in particular to sunlight.

13. Use of at least one compound of formula (I) as defined in any one of Claims 1 to 7 for the preparation of a medicament intended to prevent the harmful effects of UV radiation.

14. Use of a compound of general formula (I) as defined in any one of Claims 1 to 7 in the manufacture of a cosmetic composition as agent for protecting the skin and/or the hair against ultraviolet radiation, in particular sunlight.

15. Use of a compound of general formula (I) as defined in any one of Claims 1 to 7 in the manufacture of a cosmetic composition as agent for protecting from the variation in the colour of the skin due to UV radiation.
